# EUROPÄISCHE PATENTSCHRIFT

(11) **EP 2 252 267 B1**
(45) Veröffentlichungstag und Bekanntmachung des Hinweises auf die Patenterteilung: **28.09.2016**
(21) Anmeldenummer: 09722255.8
(22) Anmeldetag: 26.02.2009
(51) Int. Cl.: A61K 9/107, A61K 9/19, A61K 47/14, A61K 47/26

(54) **LYOPHILISIERTE NANOEMULSION**
LYOPHILIZED NANOEMULSION
NANOÉMULSION LYOPHILISÉE

(30) Priorität: 20.03.2008 DE 102008015366
(43) Veröffentlichungstag der Anmeldung: 24.11.2010
(73) Patentinhaber: Merck Patent GmbH, 64293 Darmstadt (DE)
(72) Erfinder: HANEFELD, Andrea, 69115 Heidelberg (DE); SCHMIDT, Martina Victoria, 60320 Frankfurt am Main (DE); GEISSLER, Simon, 61350 Bad Homburg (DE); LANGGUTH, Peter, 55099 Mainz (DE)
(86) Internationale Anmeldenummer: PCT/EP2009/001355
(87) Internationale Veröffentlichungsnummer: WO 2009/115175

(56) Entgegenhaltungen:
- EP-A- 0 159 237
- US-A1- 2002 193 831
- US-A1- 2003 147 959
- TOORISAKA ET AL: "An enteric-coated dry emulsion formulation for oral insulin delivery" JOURNAL OF CONTROLLED RELEASE, ELSEVIER, AMSTERDAM, NL, Bd. 107, Nr. 1, 20. September 2005 (2005-09-20), Seiten 91-96, XP005064063 ISSN: 0168-3659
- MARINA CERDEIRA ET AL: 'Microencapsulating Properties of Trehalose and of its Blends with Sucrose and Lactose' JOURNAL OF FOOD SCIENCE Bd. 70, Nr. 6, 01 August 2005, Seiten E401 - E408, XP055025792 DOI: 10.1111/j.1365-2621.2005.tb11445.x ISSN: 0022-1147

## Beschreibung

Die vorliegende Erfindung betrifft eine lyophilisierte Nanoemulsion enthaltend eine lipophile Phase und einen oder mehrere Saccharose-Fettsäureester, die aus der lyophilisierten Nanoemulsion durch Redispersion herstellbare Nanoemulsion sowie Verfahren zur Herstellung der lyophilisierten Nanoemulsion.

Emulsionen sind disperse Systeme, die aus zwei miteinander nicht mischbaren Flüssigkeiten bestehen, von denen eine die innere, dispergierte Phase, in der anderen der äußeren, geschlossenen Phase fein verteilt ist.

Nanoemulsionen sind Emulsionssysteme, in denen die innere, dispergierte, Phase aus sehr feinen lipophilen Tröpfchen in einem Größenbereich von circa 20 bis circa 500 nm besteht, die homogen in der aus Wasser bestehenden äußeren Phase verteilt vorliegen (O/W-Emulsion). Nanoemulsionen können bevorzugt parenteral angewendet werden und kommen insbesondere zur intravenösen Ernährung von Patienten, die oral keine Nahrung aufnehmen können, zum Einsatz, kommerziell erhältliche Markenprodukte sind z.B. Intralipid^{®}, Lipodfundin^{®}, Lipovenös^{®}. Alle Nanoemulsionen weisen ein milchig-trübes Aussehen auf.

Von Nanoemulsionen abzugrenzen sind Mikroemulsionen, in denen die innere, dispergierte Phase eine Partikelgröße von 10 nm bis 50 nm aufweist. Mikroemulsionen enthalten eine gegenüber Nanoemulsionen deutlich erhöhte Emulgatorkonzentration sowie meist auch einen Co-Emulgator. Mikroemulsionen sind selbstemulgierend und weisen ein klares oder opaleszierendes Aussehen auf, sind aber infolge der hohen Emulgatorkonzentration schlecht verträglich und daher nur sehr begrenzt parenteral applizierbar.

Intravenös applizierbare Nanoemulsionen stellen hohe Anforderungen an die Verträglichkeit ihrer Inhaltsstoffe und die Teilchengröße der Fettpartikel. Als Fettkomponente kommen in der parenteralen Ernährung vorzugsweise Öle mit einem hohen Anteil an ungesättigten Fettsäuren wie Sojabohnen-, Safflor- und Baumwollöl, als Emulgatoren Lecithine wie Ei-, Soja- und Cerebrallecitin zur Anwendung und enthalten zumeist ferner Antioxidantien wie Tocopherolacetat sowie ggf. weitere Hilfsstoffe.

Die Emulsionsherstellung erfolgt üblicherweise durch Voremulgierung der erwärmten Öl- und Wasserphasen mit einem Mixer, gefolgt von Feinstemulgierung mit einem Hochdruckhomogenisator und anschließender Sterilisation mit überhitztem Wasserdampf.

Nanoemulsionen können unter geringem Zeitaufwand mit Standardzerkleinerungstechniken (Hochdruckhomogenisation) hergestellt werden. Nanoemulsionen sind jedoch thermodynamisch instabil und weisen daher eine oft unzureichende Lagerstabilität auf. Bei Lagerung über längere Zeiträume, insbesondere bei erhöhten Temperaturen und Temperaturschwankungen, kommt es zu einem Ineinanderfließen der Fettpartikel (Koaleszenz), sodass die Nanoemulsion insgesamt unbrauchbar wird.

Das "Handbook on Injectable Drugs" (American Society of Hospital Pharmacists, Seite 237-244 (1986), Lawrence A. Trissel) beschreibt einige kommerziell verfügbare Zubereitungen. Sie enthalten Sojabohnenöl bzw. Saffloröl, Eilecithin, Glycerin und Wasser und haben mittlere Teilchengrößen von ≤ 0,5 µm.

Nanoemulsionen wurden auch wiederholt als Trägersysteme für parenteral zu verabreichende lipophile Arzneistoffe eingesetzt. Ziel ist es dabei, die therapeutische Wirksamkeit und Sicherheit von Arzneistoffen durch kontrollierte Freigabe aus Emulsionssystemen (Drug Delivery System) zu erhöhen.

Entsprechend ihren Löslichkeitseigenschaften sind in Nanoemulsionen enthaltene lipophile Wirkstoffe teilweise oder vollständig in die Fettpartikel eingebettet. Damit wird das pharmakokinetische Verhalten des Wirkstoffes maßgeblich durch das pharmakokinetische Verhalten der Trägerzubereitungen bestimmt, aus welcher dieser erst freigesetzt wird. Durch verzögerte Freisetzung werden hohe lokale Wirkstoffkonzentrationen vermieden, der Abbau verringert und damit die Wirkungsdauer erhöht. Ein Beispiel für eine kommerziell erhältliche, einen Wirkstoff enthaltende Nanoemulsion ist Diazepam^{®}-Lipuro.

Alle kommerziell erhältlichen Nanoemulsionen enthalten Lecithin als Emulgator. Da Lecithin hydrolyseempfindlich ist und das Hydrolyseprodukt Lysolecithin Hämolyse bewirken kann, muss der pH-Wert der Emulsionen mittels NaOH oder NaOleat eingestellt werden und/oder Hilfsstoffe zugesetzt werden, die die Hydrolyse hemmen. Allgemein sollten Hilfsstoffe, die nicht aus dem Verwendungszweck heraus begründet sind und lediglich zur Stabilisierung dienen, wann immer möglich vermieden werden, um das sich hierdurch ergebende potentielle Risiko von Schädigungen grundsätzlich auszuschließen.

JP 11171796 A beschreibt die Herstellung einer den Wirkstoff Teprenon enthaltenden Emulsion zur oralen Verabreichung, die einen Saccharose-Fettsäureester als Emulgator enthält. Ziel der beschriebenen Entwicklung ist es den oxidationsempfindlichen Wirkstoff vor Abbau zu schützen und eine wohlschmeckende Zubereitung für die orale Verabreichung zur Verfügung zu stellen. Gefriertrocknung der Emulsion wird nicht beschrieben.

US-Patent Nr. 5,750,142 beschreibt eine lyophilisierte Nanoemulsion, die auch für die parenterale Verabreichung geeignet sein soll. Neben der Fettphase und dem Emulgator enthält die lyophilisierte Emulsion mindestens 40 Gew.-% eines (Amin)-Gefrierschutzmittels. Alle Emulsionen der Ausführungsbeispiele enthalten neben Lecithin als Emulgator einen Co-Emulgator sowie α-Tocopherol als Antioxidans. Weiterhin ist zumeist Polyvinylpyrrolidon (PVP) enthalten, das als Suspendierungsmittel die Tröpfchengröße der rekonstituierten Emulsion kontrollieren und im nanoscaligen Bereich halten soll.

WO 94/14418 A1 beschreibt eine lyophilisierte Emulsionszusammensetzung, deren innere, lipophile Phase aus hydrophilen Emulgatoren und acetylierten Monoglyceriden besteht, und die weiterhin Zucker oder Zuckeralkohole als Gefrierschutzmittel/Gerüstbildner enthält. Acetylierte Monoglyceride sind amphiphil wirksam und daher aus toxikologischer Sicht nicht unbedenklich. Weiterhin enthält die Emulsionszusammensetzung hohe Anteile an Zuckern / Zuckeralkoholen (in den Ausführungsbeispielen beträgt das Gewichtsverhältnis an Zucker: Emulgator + acetylierte Monoglyceride 2 : 1), so dass durch Rekonstitution entweder hyperosmotische und damit schlecht verträgliche Emulsionen oder aber Emulsionen mit nur geringem Anteil an lipophiler Phase herstellbar sind.

JP 52-96724 beschreibt lyophilisierte O/W-Emulsionen, die neben einem Öl/Fett und Emulgatoren einen filmbildenden Hilfsstoff wie z.B. Gelatine, PVP, Methylcellulose, PVA, Polyethylenglykol oder Saccharose-Fettsäureester enthalten. Sowohl die zunächst hergestellten Ausgangsemulsionen (vor Gefriertrocknung) als auch die aus der lyophilisierten Emulsion redispergierten Emulsionen weisen Tröpfchengrößen im zweistelligen µm-Bereich auf. Weiterhin ergibt sich gegenüber der Ausgangsemulsion nach Gefriertrocknung und Redispersion eine deutliche Erhöhung der Partikelgröße. Die Emulsionen sind nicht für die parenterale Verabreichung geeignet.

JP 2004/161650 A beschreibt eine gefriergetrocknete W/O/W-Emulsion mit Polyglycerol-Fettsäureester oder Saccharose-Fettsäureester als Emulgator, deren äußere Phase Polyvinylalcohol oder Xantangummi und einen Zucker enthält. Ohne Polyvinylalcohol oder Xantangummi sind die lyophilisierten W/O/W-Emulsionen nicht zu Emulsionen redispergierbar, nach Redispersion der diese Stoffe enthaltenden Emulsionen ergeben sich W/O/W-Emulsionen mit mittleren Partikelgrößen von circa 500 bis > 3000 nm. Die redispergierten W/O/W-Emulsionen sind für die orale, nicht aber für die parenterale Verabreichung geeignet.

Die Arbeit von Cereida et al. (Microencapsulating Properties of Trehalose and of its Blends with Sucrose and Lactose", J Food Sci 70 (2005) E401-E408) beschreibt eine Mikroemulsion hergestellt aus niedrigschmelzendes Milchfett und Palmitinsäure Saccharoseester. Die lyophilisierte Emulsion enthält Lipid und Saccharose-Fettsäureester in einem Gewichtsverhältnis von 11:1 und Trehalose als Gefrierschutzmittel.

Eine kürzlich im Internet veröffentlichte Arbeit (Dong Zhao et al.: A submicron emulsion for intravenous injection: Characerization, in vitro and in vivo antitumor effect, Int J Pharm (2007), doi:10.1016/j.ijpharm.2008.01.055) offenbart einen gefriergetrocknete Nanoemulsion, die neben einem Öl und Lecithin als Emulgator einen hohen Anteil an Saccharose enthält (Gewichtsverhältnis Ölphase : Zucker = 1 : 5). Aufgrund der Verwendung von hydrolyseempfindlichem Lecithin enthält die Emulsion zusätzlich Vitamin E. Der hohe Anteil an Saccharose kann nachteilhaft nach Redispersion zu hypertonen Lösungen führen. Weiterhin ist der Zuckeranteil bei Verabreichung an Diabetiker nicht unkritisch und kann zu hyperosmotischen Formulierungen führen.

Aufgabe der vorliegenden Erfindung war, den Nachteilen des Standes der Technik abzuhelfen und eine Nanoemulsion bereitzustellen, die lyophilisierbar ist und durch Redispersion mit Wasser zu einer parenteral verabreichbaren Nanoemulsion mit einer der Ausgangsemulsion entsprechenden Tröpfchengrößenverteilung redispergierbar ist. Die lyophilisierte Nanoemulsion sollte möglichst einfach aufgebaut sein und auch ohne den Zusatz von weiteren Stoffen wie Gefrierschutzmitteln und Antioxidantien herstellbar und über lange Zeiträume (Haltbarkeit 3 Jahre) stabil lagerfähig sein.

Überraschenderweise wurde gefunden, dass eine diesen Anforderungen entsprechende lyophilisierte Nanoemulsion bereitgestellt werden kann, wenn diese neben einem Lipid Saccharose-Fettsäureester als Emulgatoren enthält. Gegenstand der Erfindung ist daher eine lyophilisierte Nanoemulsion, die dadurch gekennzeichnet ist, dass diese mindestens ein Lipid und mindestens einen Saccharose -Fettsäureester enthält und Lipid und Saccharose-Fettsäureester zueinander in einem Gewichtsverhältnis von 1 : 1 bis 5 : 1 vorliegen.

Aufgrund der Stabilität der Saccharose-Fettsäureester ist vorteilhaft weder eine pH-Werteinstellung (Alkalisierung) noch der Zusatz eines Antioxidans erforderlich. Überraschenderweise ist die lyophilisierte Nanoemulsion auch ohne Zusatz von Gefrierschutzmitteln herstellbar und kann dennoch durch Zusatz einer wässrigen Flüssigkeit zu einer Nanoemulsion redispergiert werden, deren Teilchengrößenverteilung weit gehend der der Ausgangsemulsion entspricht. Infolge des möglichen Verzichts auf Gefrierschutzmittel verringert sich das mikrobielle Kontaminationsrisiko (die Zusatzstoffe fördern den mikrobiellen Wachstum). Demgemäß enthält die erfindungsgemäße Nanoemulsion nach einer bevorzugten Ausführungsform keine Gefrierschutzmittel wie z.B. Zucker, Zuckeralkohole oder Aminosäuren.

Weiterhin vorteilhaft ist, dass die lyophilisierte Nanoemulsion (durch Zugabe von wenig Wasser) auch zu einer Nanoemulsion redispergierbar ist, die einen gegenüber der Ausgangsemulsion erhöhten Anteil an innerer (Lipid)-Phase aufweist, ohne dass dies mit einer erhöhten Osmolalität einhergeht, die deren parenterale Verabreichbarkeit insgesamt infrage stellt (hyperosmotische Nanoemulsionen sind parenteral schlecht verträglich).

Durch Einarbeitung von lipophilen Wirkstoffen kann die erfindungsgemäße lyophilisierte Nanoemulsion vorteilhaft auch als Drug Delivery System eingesetzt werden, das nach Redispersion mit einer wässrigen Flüssigkeit parenteral oder auch oral verabreichbar ist. Daneben ist die lyophilisierte Nanoemulsion beispielsweise auch als Trägeremulsion für die parenterale Fettsubstitutionstherapie geeignet.

Nach einer Ausführungsform der Erfindung enthält die lyophilisierte Nanoemulsion einen oder mehrere Wirkstoffe. Gegenstand der Erfindung ist daher auch eine lyophilisierte Nanoemulsion, die dadurch gekennzeichnet ist, dass sie mindestens ein Lipid, mindestens einen Saccharose-Fettsäureester und mindestens einen Wirkstoff enthält.

Bevorzugt sind lipophile Wirkstoffe, das heißt Substanzen, die relativ unlöslich in Wasser aber löslich in einem oder mehreren fetten Lösungsmitteln wie z.B. Benzol, Chloroform, Aceton, Äther oder Hexan sind. Bevorzugt sind pharmazeutisch wirksame Substanzen, die in Fetten/Ölen (Triester des dreiwertigen Alkohols Glycerol mit gesättigten/ungesättigten Monocarbonsäuren unterschiedlicher Kettenlänge) eine Löslichkeit von bevorzugt >1µg/ml und ein Öl-/Wasserverteilungsverhältnis von >1:1 aufweisen. Beispiele für lipophile Wirkstoffe, die in der erfindungsgemäßen lyophilisierte Nanoemulsion enthalten sein können, (Fibrate, z.B. Fenofibrat/Clofibrat, Benzodiazepine, z.B. Carbamazepin, Azole, z.B. Bifonazol, Steroide, z.B. Danazol).

Die lyophilisierte Nanoemulsion kann mit einer wässrigen Flüssigkeit, bevorzugt Wasser, zu einer Nanoemulsion redispergiert werden, deren Teilchengrößenverteilung weitgehend der Teilchengrößenverteilung der Ausgangsemulsion (d. h. der Nanoemulsion vor Überführung zur lyophilisierte Nanoemulsion mittels Gefriertrocknung), entspricht. Neben reinem Wasser, insbesondere Wasser zur Injektion, kann die zur Redispersion verwendete wässrige Flüssigkeit auch gelöste Stoffe, beispielsweise Isotonisierungsmittel wie Natriumchlorid oder Dextrose enthalten. Zur Redispersion besonders geeignete wässrige Flüssigkeiten sind physiologische Natriumchlorid-Lösung und Dextrose-Lösungen.

Die erfindungsgemäße lyophilisierte Emulsion kann als Lipid grundsätzlich alle Lipide enthalten, die aus pharmazeutischer Sicht zur Herstellung von Nanoemulsionen geeignet sind, insbesondere Mono-, Di- und/oder Triglyceride mit C₈- bis C₂₂-Fettsäuren, besonders bevorzugt C₈- bis C₁₈-Fettsäuren, und/oder fettlösliche Vitamine. Gegenstand der Erfindung ist daher auch eine lyophilisierte Nanoemulsion, die dadurch gekennzeichnet ist, dass als Lipid Mono-, Di- und/oder Triglyceride mit C₈- bis C₂₂-Fettsäuren, bevorzugt C₈- bis C₁₈-Fettsäuren, ungesättigte oder gesättigte C₈- bis C₂₂-Fettsäuren und/oder fettlösliche Vitamine oder fettlösliche Wirkstoffe , vorzugsweise, enthalten sind.

Beispiele geeigneter Lipide sind natürliche Öle, wie zum Beispiel Erdnussöl, Mandelöl, Olivenöl, Sesamöl, Sojabohnenöl, Distelöl (Saffloröl) oder Baumwollöl, halbsynthetische Öle wie z.B. Mittelkettige Triglyceride (MCT), ein Triglyceridgemisch, das als Fettsäuren hauptsächlich C₈- bis C₁₂-Fettsäuren, insbesondere Caprylsäure und Caprinsäure, enthält, aber auch die fettlöslichen Vitamine (Vitamin A, Vitamin D, Vitamin E, Vitamin K), wovon Vitamin E und Vitamin D bevorzugt sind.

Bevorzugt enthält die lyophilisierte Nanoemulsion als Lipid Triglyceride. Als Triglyceride besonders bevorzugt sind Triglyceride von C₈- bis C₁₂-Fettsäuren. Saccharose-Fettsäureester sind Ester, die z.B. durch Umesterung von Saccharose mit Methylestern langkettiger Fettsäuren erhalten werden können. Erfindungsgemäß einsetzbar sind Ester der Saccharose mit C₈- bis C₂₂-Fettsäuren, bevorzugt sind Saccharoseester mit C₁₂- bis C₁₈-Fettsäuren, insbesondere Laurin-, Myristin-, Palmitin- und Stearinsäure, besonders bevorzugt sind Laurin- und Myristinsäure. Saccharose-Fettsäureester sind auch kommerziell verfügbar und werden beispielsweise von der Firma Mitsubishi Kagaku Corp., Tokio, Japan unter der Handlungsbezeichnung Ryoto vertrieben. Beispiele hierfür sind Saccharose-Fettsäureester mit Laurinsäure, mit Myristinsäure oder mit Stearinsäure, die die Handelbezeichnungen Ryoto L 1695, Ryoto M 1695 und Ryoto S 1670 aufweisen.

In der lyophilisierten Nanoemulsion liegen das hierin enthaltene Lipid und die Saccharose-Fettsäureester zueinander in einem Gewichtsverhältnis von 1 : 1 bis 5 : 1, bevorzugt in einem Gewichtsverhältnis von 2 : 1 bis 4 : 1 vor. Gegenstand der Erfindung ist daher eine lyophilisierte Nanoemulsion, die dadurch gekennzeichnet ist, dass Lipid und Saccharose-Fettsäureester zueinander in einem Gewichtsverhältnis von 1 : 1 bis 5 : 1, bevorzugt in einem Gewichtsverhältnis von 2 : 1 bis 4 : 1 vorliegen.

Die erfindungsgemäße lyophilisierte Nanoemulsion kann durch Gefriertrocknung einer Nanoemulsion hergestellt werden, worin Lipid zusammen mit dem Emulgator in einer wässrigen Phase dispergiert vorliegen. Zweckmäßigerweise liegen Lipid und wässrige Phase in der zu lyophilisierenden Nanoemulsion (Ausgangsemulsion) zueinander in einem Gewichtsverhältnis von 0,5 : 99,5 bis 30 : 70 vor. Gegenstand der Erfindung ist daher auch eine lyophilisierte Nanoemulsion, die dadurch gekennzeichnet ist, dass diese durch Gefriertrocknung einer Nanoemulsion hergestellt wird, in welcher Lipid und wässrige Phase zueinander in einem Gewichtsverhältnis von 0,5 : 99,5 bis 30 : 70 vorliegen.

Die lyophilisierte Nanoemulsion kann in einfacher Weise durch Zugabe einer wässrigen Flüssigkeit zu einer Nanoemulsion redispergiert werden, die direkt, beispielsweise parenteral oder oral, verabreichbar ist. Gegenstand der Erfindung ist daher auch eine Nanoemulsion, die dadurch gekennzeichnet ist, dass diese aus der lyophilisierten Nanoemulsion durch Redispersion mit einer wässrigen Flüssigkeit hergestellt wird.

In Abhängigkeit von der zur Redispersion der lyophilisierten Emulsion zugegebenen Menge an wässriger Flüssigkeit kann in der durch Redispersion entstehenden Nanoemulsion das Verhältnis von Lipid und wässriger Flüssigkeit zueinander über weite Bereiche variiert werden. Wird z.B. wenig wässrige Flüssigkeit verwendet, entstehen hochkonzentrierte Nanoemulsionen, die bei Wirkstoffbeladung pro Volumeneinheit hohe Wirkstoffmengen enthalten. Dies ermöglicht es auch große Wirkstoffmengen mittels kleiner Volumina zu verabreichen. Sind also hohe Wirkstoffdosen zu verabreichen, kann das hierzu erforderliche Injektionsvolumen vorteilhaft deutlich reduziert werden und beispielsweise eine intravenöse Infusion durch eine intravenöse Injektion ersetzt werden. Da die lyophilisierte Nanoemulsion auch kein Gefrierschutzmittel, wie z.B. Zucker, erfordert, kann deren Redispersion auch mit wenig wässriger Flüssigkeit erfolgen, ohne dass diese, wie die lyophilisierten Emulsionen des Standes der Technik, zwangsläufig hyperton werden, was, beispielsweise bei parenteraler Verabreichung, zu den bekannten Verträglichkeitsproblemen führt.

Gegenstand der Erfindung ist daher auch einen Nanoemulsion, die dadurch gekennzeichnet ist, dass hierin Lipid und Wasser zueinander in einem Gewichtsverhältnis von 0,5 : 99,5 bis 50 : 50, vorzugsweise in einem Gewichtsverhältnis von 5 : 95 bis 50 : 50 vorliegen.

Die erfindungsgemäße lyophilisierte Nanoemulsion kann hergestellt werden, indem einer mittels der in der Arzneimittelherstellung gebräuchlichen Verfahren und Technologien hergestellten Emulsion die wässrige Phase durch Lyophilisation entfernt wird. Gegenstand der Erfindung ist daher auch ein Verfahren zur Herstellung der erfindungsgemäßen lyophilisierten Nanoemulsion, welches dadurch gekennzeichnet ist, dass zunächst in üblicher Weise eine Nanoemulsion hergestellt wird, und dieser anschließend mittels Gefriertrocknung die äußere, wässrige Phase entzogen wird.

Sollen in die erfindungsgemäße lyophilisierte Nanoemulsion Wirkstoffe eingearbeitet werden, kann dies bei hydrophilen Wirkstoffen durch Lösen in der wässrigen Tensidphase (Emulgatorgrenzschicht und Emulgatormizellen) und bei lipophilen Wirkstoffen durch Lösen in der Emulgator und Lipid enthaltenden Phase erfolgen. Alternativ kann die Wirkstoffzugabe auch unmittelbar vor Durchführung der Lyophilisation erfolgen, was insbesondere für hydrolyseempfindliche und/oder thermolabile Wirkstoffe vorteilhaft ist. Damit kann das Verfahren zur Herstellung der erfindungsgemäßen wirkstoffhaltigen Emulsionszusammensetzung in vorteilhafter Weise an die physikochemischen Eigenschaften der Wirkstoffe angepasst werden. Daher wird in einer zweckmäßigen Ausführungsform des erfindungsgemäßen Verfahrens mindestens ein Wirkstoff vor Emulgierung entweder in der wässrigen Phase oder in der Emulgator und Lipid enthaltenden Phase gelöst oder mindestens ein Wirkstoff der Emulsion vor ihrer Gefriertrocknung zugegeben.

Hierbei ist zu beachten, dass die Wirkstoffverteilung innerhalb des dispersen Systems vor Lyophilisation gewährleistet ist, was mit üblichen Methoden wie Gleichgewichtsdialyse, Differentialdialyse und Ultrafiltration leicht geprüft werden kann.

Die Beispiele, ohne hierauf beschränkt zu sein, erläutern die Erfindung.

### Beispiele:

Allgemeines Verfahren zur Herstellung der Nanoemulsionen Der Sucroseester wird bei 50°C in H₂O gelöst, das Öl auf 50°C temperiert, Wasser- und Ölphase miteinander vereinigt, die vereinigten Phasen mittels Ultraturrax bei 8000 rpm für 3 Minuten vorhomogenisiert und anschließend in einem Avestin Emulsiflex-C3 einer Hochdruckhomogenisation unterzogen (8 Zyklen, 4x1000bar + 4x2000bar).

Die Nanoemulsion wird anschließend folgendem Gefriertrocknungsprozess unterzogen:
1) Einfrierprozess: -50°C, 3,5 Stunden
2) Haupttrocknung: -40°C, geeigneter Unterdruck über bis zu 48 Stunden
3) Nachtrocknung: -40 - +20°C, geeigneter Unterdruck über bis zu 12 Stunden.

### Beispiel 1

Unter Verwendung der nachfolgenden angeführten Stoffe wird gemäß dem oben beschriebenen allgemeinen Verfahren eine Nanoemulsion hergestellt.

| | |
|---|---|
| Saccharose- Laurinsäureester (Ryoto L1695) | 5g |
| Mittelkettige Triglyceride | 10g |
| Wasser | 85g |

### Beispiel 2:

Unter Verwendung der nachfolgenden angeführten Stoffe wird gemäß dem oben beschriebenen allgemeinen Verfahren eine Nanoemulsion hergestellt.

| | |
|---|---|
| Saccharose- Laurinsäureester (Ryoto L1695) | 5g |
| Sojaöl | 10g |
| Wasser | 85g |

### Beispiel 3

Unter Verwendung der nachfolgenden angeführten Stoffe wird gemäß dem oben beschriebenen allgemeinen Verfahren eine Nanoemulsion hergestellt.

| | |
|---|---|
| Saccharose-Myristinsäureester (Ryoto M1695) | 5g |
| Mittelkettige Triglyceride | 10g |
| Wasser | 85g |

### Beispiel 4

Unter Verwendung der nachfolgenden angeführten Stoffe wird gemäß dem oben beschriebenen allgemeinen Verfahren eine Nanoemulsion hergestellt.

| | |
|---|---|
| Saccharose-Laurinsäureester (Ryoto L1695) | 5g |
| Mittelkettige Triglyceride | 20g |
| Wasser | 75g |

### Beispiel 5

Unter Verwendung der nachfolgenden angeführten Stoffe werden gemäß dem oben beschriebenen allgemeinen Verfahren eine Nanoemulsion hergestellt.

| | |
|---|---|
| Saccharose-Stearinsäureester (Ryoto S1670) | 5g |
| Mittelkettige Triglyceride | 20g |
| Wasser | 75g |

### Beispiel 6

Unter Verwendung der nachfolgenden angeführten Stoffe wird gemäß dem oben beschriebenen allgemeinen Verfahren eine Nanoemulsion hergestellt.

| | |
|---|---|
| Saccharose-Laurinsäureester (Ryoto L1695) | 5g |
| Mittelkettige Triglyceride | 30g |
| Wasser | 65g |

### Beispiel 7

Unter Verwendung der nachfolgenden angeführten Stoffe wird gemäß dem oben beschriebenen allgemeinen Verfahren eine Nanoemulsion hergestellt.

| | |
|---|---|
| Saccharose-Laurinsäureester (Ryoto L1695) | 7.5g |
| Mittelkettige Triglyceride | 30g |
| Wasser | 62.5g |

### Beispiel 8

Unter Verwendung der nachfolgenden angeführten Stoffe wird gemäß dem oben beschriebenen allgemeinen Verfahren eine Nanoemulsion hergestellt.

| | |
|---|---|
| Saccharose-Laurinsäureester (Ryoto L1695) | 10g |
| Mittelkettige Triglyceride | 30g |
| Wasser | 60g |

### Beispiel 9

Unter Verwendung der nachfolgenden angeführten Stoffe wird gemäß dem oben beschriebenen allgemeinen Verfahren eine Nanoemulsion hergestellt.

| | |
|---|---|
| Saccharose-Laurinsäureester (Ryoto L1695) | 7.5g |
| Mittelkettige Triglyceride | 10g |
| Wasser | 82.5g |

### Beispiel 10

Unter Verwendung der nachfolgenden angeführten Stoffe wird gemäß dem oben beschriebenen allgemeinen Verfahren eine Nanoemulsion hergestellt.

| | |
|---|---|
| Saccharose-Laurinsäureester (Ryoto L1695) | 10g |
| Mittelkettige Triglyceride | 10g |
| Wasser | 80g |

### Beispiel 11

Unter Verwendung der nachfolgenden angeführten Stoffe wird gemäß dem oben beschriebenen allgemeinen Verfahren eine Nanoemulsion hergestellt.

| | |
|---|---|
| Saccharose-Laurinsäureester (Ryoto M1695) | 7.5g |
| Mittelkettige Triglyceride | 10g |
| Wasser | 82.5g |

### Beispiel 12

Unter Verwendung der nachfolgenden angeführten Stoffe wird gemäß dem oben beschriebenen allgemeinen Verfahren eine Nanoemulsion hergestellt.

| | |
|---|---|
| Saccharose-Laurinsäureester (Ryoto M1695) | 10g |
| Mittelkettige Triglyceride | 10g |
| Wasser | 80g |

### Beispiel 13 (wirkstoffhaltige Nanoemulsion)

Unter Verwendung der nachfolgenden angeführten Stoffe wird gemäß dem oben beschriebenen allgemeinen Verfahren eine Nanoemulsion hergestellt.

| | |
|---|---|
| Saccharose-Laurinsäureester (Ryoto L1695) | 7.5g |
| Mittelkettige Triglyceride | 30g |
| Fenofibrat | 1.5g |
| Wasser | 61g |

### Vergleichsbeispiel 1

Unter Verwendung der nachfolgenden angeführten Stoffe wird gemäß dem oben beschriebenen allgemeinen Verfahren eine Nanoemulsion hergestellt.

| | |
|---|---|
| PEG660-12-Hydroxystearat (Solutol HS15) | 5g |
| Mittelkettige Triglyceride | 10g |
| Wasser | 85g |

### Vergleichsbeispiel 2

Unter Verwendung der nachfolgenden angeführten Stoffe wird gemäß dem oben beschriebenen allgemeinen Verfahren eine Nanoemulsion hergestellt.

| | |
|---|---|
| Phosphatidylcholin, auf Sojabasis, 75% Reinheit): | 5g |
| Sojaöl | 10g |
| Wasser | 85g |

Analog dem oben beschriebenen allgemeinen Verfahren werden Nanoemulsionen mit Solutol ® HS 15 (PEG660-12-Hydroxystearat) / Cremophor ® RH 40 (PEG-40 hydriertes Rizinusöl) / Lipoid S75 (sojabasiertes Phosphatidylcholin) und Lipoid E80 (eibasiertes Phosphatidylcholin) als Emulgatoren und Mittelkettigen Triglyceriden (MCT) als Lipid hergestellt (Gewichtsanteile siehe Tab. 2) und gefriergetrocknet. Zusätzlich hierzu wird eine kommerziell erhältliche Nanoemulsion (Lipovenös®, enthaltend Phosphatidylcholin als Emulgator) unter den gleichen Bedingungen lyophilisiert.

Keines der erhaltenen Lyophilisate (lyophilisierten Nanoemulsionen) weist einen intakten Lyophilisationskuchen auf, alle sind kollabiert. Bei Redispersion mit Wasser ergibt sich Phasenseparation bzw. werden sehr grobe Emulsionen gebildet, die nicht zur parenteralen Applikation geeignet sind.

Im Gegensatz dazu zeigen die Saccharose-Fettsäureester-haltigen Nanoemulsionen eine strukturerhaltende Kuchenbildung nach Lyophilisation. Bei Wasseraddition zum Lyophilisationskuchen bilden sich ohne Energieeintrag Nanoemulsionen aus, die Partikelgrößen im Bereich <200nm aufweisen.

Die Größenmessungen werden mittels Photonenkorrelationsspektroskopie vorgenommen. Bestimmt wird der intensitätsgewichtete mittlere Durchmesser der Emulsionströpfchen an einem Zeatasizer Nano ZS (Malvern Ltd, UK). Die Auswertung der Streusignale erfolgt über die Software DTS 5.03 (Malvern Ltd. UK). Als Maß für die Größenverteilung der Probe ist in dieser Software der Polydispersitätsindex (PDI) implementiert.

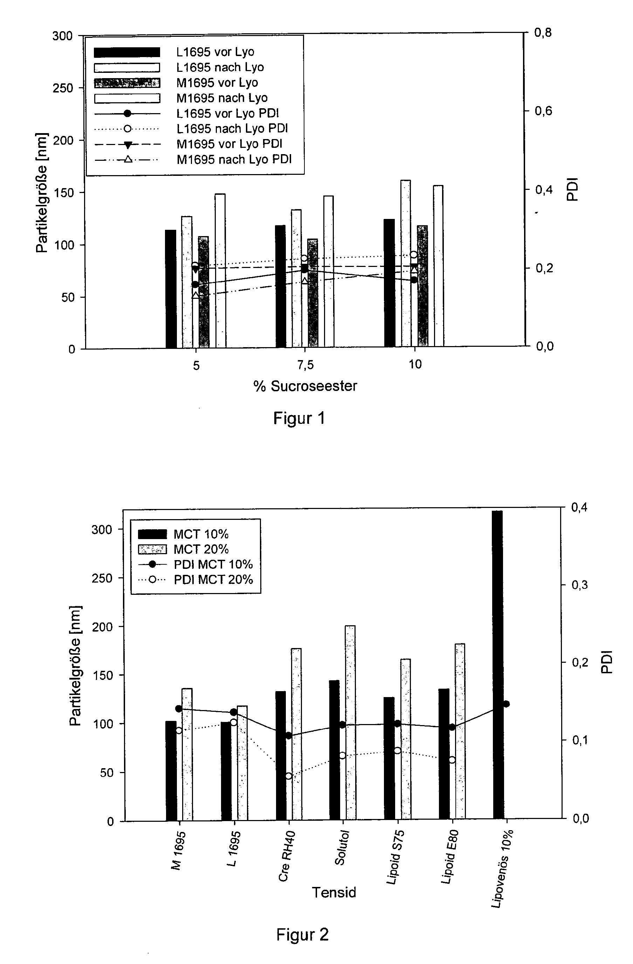

Die mittleren Partikelgrößen und der Polydispersitätsindex (PDI) der Saccharose-Fettsäureester enthaltenden Emulsionen vor und nach Gefriertrocknung sind in Tabelle 1 zusammengestellt und in Figur 1 graphisch dargestellt.

**Tabelle 1**

| **Beispiel** | **Emulgator** | **Lipid** | **vor Gefriertrocknung** | | **nach Gefriertrocknung** | |
|---|---|---|---|---|---|---|
| **Nr**. | **Ryoto L1695 [%]** | **MCT [%]** | **Partikelgröße [nm]** | **PDI** | **Partikelgröße [nm]** | **PDI** |
| 1 | 5 | 10 | 113,4 | 0,162 | 126,2 | 0,21 |
| 9 | 7,5 | 10 | 117,1 | 0,198 | 132,1 | 0,227 |
| 10 | 10 | 10 | 122,2 | 0,17 | 159,7 | 0,235 |
| | | | | | | |

| | **Emulgator** | **Lipid** | **vor Gefriertrocknung** | | **nach Gefriertrocknung** | |
|---|---|---|---|---|---|---|
| | **Ryoto M1695 [%]** | **MCT [%]** | **Partikelgröße [nm]** | **PDI** | **Partikelgröße [nm]** | **PDI** |
| 3 | 5 | 10 | 106,9 | 0,204 | 147,5 | 0,133 |
| 11 | 7,5 | 10 | 103,8 | 0,207 | 144,9 | 0,168 |
| 12 | 10 | 10 | 116 | 0,206 | 154,6 | 0,195 |

Figur 2 und Tabelle 2 zeigen die Partikelgrößen von repräsentativen Nanoemulsionen vor Lyophilisation auf Grundlage der vorliegenden Erfindung im Vergleich zu Standardemulsionen. Überraschenderweise führen Saccharose-Fettsäurester bei gleichem Energieeintrag und gleicher Tensidkonzentration zu bis zu ∼50nm kleineren Emulsionströpfchen als die Emulgatoren, die üblicherweise für Nanoemulsionen verwendet werden.

**Tabelle 2**

| | **MCT 10%** | | **MCT 20%** | |
|---|---|---|---|---|
| **Tensid/Produkt** | **Größe [nm]** | **PDI** | **Größe [nm]** | **PDI** |
| **Ryoto M1695 2%** | 101,9 | 0,143 | 135,3 | 0,115 |
| **Ryoto L 1695 2%** | 100,6 | 0,138 | 117 | 0,125 |
| **Cremophor RH 40 2%** | 131,7 | 0,107 | 175,8 | 0,056 |
| **Solutol HS 15 2%** | 142,7 | 0,121 | 199 | 0,081 |
| **Lipoid S75** | 125 | 0,122 | 163,9 | 0,087 |
| **Lipoid E80** | 133,1 | 0,117 | 179,6 | 0,075 |
| **Lipovenös® 10%** | 316,2 | 0,146 | - | |

## Patentansprüche

1. Lyophilisierte Nanoemulsion, **dadurch gekennzeichnet, dass** diese mindestens ein Lipid und mindestens einen Saccharose-Fettsäureester enthält und Lipid und Saccharose-Fettsäureester zueinander in einem Gewichtsverhältnis von 1 : 1 bis 5 : 1 vorliegen.

2. Lyophilisierte Nanoemulsion nach Anspruch 1, **dadurch gekennzeichnet, dass** diese mindestens einen Wirkstoff enthält.

3. Lyophilisierte Nanoemulsion nach Anspruch 1 und/oder 2, **dadurch gekennzeichnet, dass** als Lipid Mono-, Di- und/oder Triglyceride mit C₈- bis C₂₂-Fettsäuren, bevorzugt C₈- bis C₁₈-Fettsäuren, und/oder fettlösliche Vitamine enthalten sind.

4. Lyophilisierte Nanoemulsion nach Anspruch 3, **dadurch gekennzeichnet, dass** als Lipid Triglyceride enthalten sind.

5. Lyophilisierte Nanoemulsionen nach Anspruch 4, **dadurch gekennzeichnet, dass** die Triglyceride C₈- bis C₁₂-Fettsäuren enthalten.

6. Lyophilisierte Nanoemulsion nach einem oder mehreren der Ansprüche 1 bis 5, **dadurch gekennzeichnet, dass** die Saccharose-Fettsäureester C₈- bis C₂₂-Fettsäuren, bevorzugt C₁₂- bis C₁₈-Fettsäuren, enthalten.

7. Lyophilisierte Nanoemulsion nach einem oder mehreren der Ansprüche 1 bis 6, **dadurch gekennzeichnet, dass** Lipid und Saccharose-Fettsäureester zueinander in einem Gewichtsverhältnis von 2 : 1 bis 4 : 1 vorliegen.

8. Lyophilisierte Nanoemulsion nach einem oder mehreren der Ansprüche 1 bis 7, **dadurch gekennzeichnet, dass** diese durch Gefriertrocknung einer Nanoemulsion hergestellt wird, in welcher Lipid und wässrige Phase in einem Gewichtsverhältnis von 0,5 : 99,5 bis 30 : 70 vorliegen.

9. Verfahren zur Herstellung der lyophilisierten Nanoemulsion nach einem oder mehreren der Ansprüche 1 bis 8, **dadurch gekennzeichnet, dass** zunächst in üblicher Weise eine Nanoemulsion hergestellt wird, und dieser anschließend mittels Gefriertrocknung die äußere, wässrige Phase entzogen wird.

10. Verfahren nach Anspruch 9, **dadurch gekennzeichnet, dass** mindestens ein Wirkstoff vor Emulgierung entweder in der wässrigen Phase oder in der Lipid enthaltenden Phase gelöst oder mindestens ein Wirkstoff der Emulsion vor ihrer Gefriertrocknung zugegeben wird.

## Claims

1. Lyophilised nanoemulsion, **characterised in that** it comprises at least one lipid and at least one sucrose fatty acid ester and lipid and sucrose fatty acid ester are present in a weight ratio to one another of 1 : 1 to 5 : 1.

2. Lyophilised nanoemulsion according to Claim 1, **characterised in that** it comprises at least one active compound.

3. Lyophilised nanoemulsion according to Claim 1 and/or 2, **characterised in that** the lipid present comprises mono-, di- and/or triglycerides with C₈- to C₂₂-fatty acids, preferably C₈- to C₁₈-fatty acids, and/or fat-soluble vitamins.

4. Lyophilised nanoemulsion according to Claim 3, **characterised in that** the lipid present comprises triglycerides.

5. Lyophilised nanoemulsions according to Claim 4, **characterised in that** the triglycerides comprise C₈- to C₁₂-fatty acids.

6. Lyophilised nanoemulsion according to one or more of Claims 1 to 5, **characterised in that** the sucrose fatty acid esters comprise C₈- to C₂₂-fatty acids, preferably C₁₂- to C₁₈-fatty acids.

7. Lyophilised nanoemulsion according to one or more of Claims 1 to 6, **characterised in that** lipid and sucrose fatty acid ester are present in a weight ratio to one another of 2 : 1 to 4 : 1.

8. Lyophilised nanoemulsion according to one or more of Claims 1 to 7, **characterised in that** it is prepared by freeze-drying a nanoemulsion in which lipid and aqueous phase are present in a weight ratio of 0.5 : 99.5 to 30 : 70.

9. Process for the preparation of the lyophilised nanoemulsion according to one or more of Claims 1 to 8, **characterised in that** firstly a nanoemulsion is prepared in a conventional manner, and the external, aqueous phase is subsequently removed therefrom by means of freeze-drying.

10. Process according to Claim 9, **characterised in that** at least one active compound is dissolved, before emulsification, either in the aqueous phase or in the lipid-containing phase or at least one active compound is added to the emulsion before freeze-drying thereof.

## Revendications

1. Nano-émulsion lyophilisée, **caractérisée en ce qu'**elle comprend au moins un lipide et au moins un ester d'acide gras de sucrose, et le lipide et l'ester d'acide gras de sucrose sont chacun présents selon un rapport en poids de l'un par rapport à l'autre de 1 : 1 à 5 : 1.

2. Nano-émulsion lyophilisée selon la revendication 1, **caractérisée en ce qu'**elle comprend au moins un composé actif.

3. Nano-émulsion lyophilisée selon les/la revendication(s) 1 et/ou 2, **caractérisée en ce que** le lipide présent comprend des monoglycérides, des diglycérides et/ou des triglycérides avec des acides gras C₈- à C₂₂-, de façon préférable des acides gras C₈- à C₁₈-, et/ou des vitamines liposolubles.

4. Nano-émulsion lyophilisée selon la revendication 3, **caractérisée en ce que** le lipide présent comprend des triglycérides.

5. Nano-émulsion lyophilisée selon la revendication 4, **caractérisée en ce que** les triglycérides comprennent des acides gras C₈- à C₁₂.

6. Nano-émulsion lyophilisée selon une ou plusieurs des revendications 1 à 5, **caractérisée en ce que** les esters d'acides gras de sucrose comprennent des acides gras C₈- à C₂₂-, de façon préférable des acides gras C₁₂-à C₁₈₋.

7. Nano-émulsion lyophilisée selon une ou plusieurs des revendications 1 à 6, **caractérisée en ce que** le lipide et l'ester d'acide gras de sucrose sont présents selon un rapport en poids de l'un par rapport à l'autre de 2 : 1 à 4 : 1.

8. Nano-émulsion lyophilisée selon une ou plusieurs des revendications 1 à 7, **caractérisée en ce qu'**elle est préparée par cryodessiccation d'une nano-émulsion dans laquelle un lipide et une phase aqueuse sont présents selon un rapport en poids de 0,5 : 99,5 à 30 : 70.

9. Procédé pour la préparation de la nano-émulsion lyophilisée selon une ou plusieurs des revendications 1 à 8, **caractérisé en ce que**, en premier lieu, une nano-émulsion est préparée d'une manière classique, et la phase aqueuse externe en est ensuite enlevée au moyen d'une cryodessiccation.

10. Procédé selon la revendication 9, **caractérisé en ce qu'**au moins un composé actif est dissout, avant émulsification, soit dans la phase aqueuse, soit dans la phase contenant un lipide ou au moins un composé actif est ajouté à l'émulsion avant sa cryodessiccation.
